# EUROPEAN PATENT SPECIFICATION

(11) **EP 4 031 099 B1**
(45) Date of publication and mention of the grant of the patent: **01.03.2023**
(21) Application number: 20775601.6
(22) Date of filing: 17.09.2020
(51) Int. Cl.: A61K 8/34, A61K 8/41, A61K 8/9789, A61Q 15/00

(54) **ANTIPERSPIRANT COMPOSITIONS**
SCHWEISSHEMMENDE ZUSAMMENSETZUNGEN
COMPOSITIONS ANTITRANSPIRANTES

(30) Priority: 20.09.2019 EP 19198703
(43) Date of publication of application: 27.07.2022
(73) Proprietor: Unilever IP Holdings B.V., 3013 AL Rotterdam (NL); Unilever Global IP Limited, Wirral, Merseyside CH62 4ZD (GB)
(72) Inventor: EVANS, Richard, Livesey, Wirral Merseyside CH63 3JW (GB)
(74) Representative: Corsten, Michael Allan
(86) International application number: PCT/EP2020/076016
(87) International publication number: WO 2021/053093

(56) References cited:
- EP-A2- 1 547 576
- WO-A1-02/11690
- WO-A2-2009/087578
- CN-A- 108 236 646
- US-A- 6 022 531
- US-A1- 2017 137 366

## Description

### Field of Invention

The present invention is in the field of antiperspirancy, in particular methods of achieving antiperspirancy without gland blockage by the use of natural antiperspirant actives.

### Background

There have been several publications published relating to the reduction of sweating using actives that do not block the sweat glands or pores.

WO 02/011690 (Unilever) discloses an antiperspirancy method whereby calcium channels with the secretory coil cells of the eccrine glands are blocked, thereby controlling sweat production at its source.

US 8,618,160 B2 (Rose U) discloses wipes containing glycopyrrolate, a muscarinic anticholinergic, as a treatment for hyperhidrosis.

Certain anticholinergic drugs have also been used to treat hyperhidrosis, with varying degrees of success. These drugs include Ditropan^{®} (oxybutynin), Probanthine^{®} (propantheline bromide) and Cogentin^{®} (benzotropine).

US 2008/0207737 (Zinger) discloses a topical composition for antiperspirant benefit comprising varying levels of oxybutynin.

### Summary of invention

It is an object of the invention to reduce perspiration on the surface of the human body by the use a non-pore blocking antiperspirant agent, in particular by the use of such an agent in a composition not comprising any pore blocking antiperspirant active.

It is a further object of the invention to reduce perspiration on the surface of the human body by the use an antiperspirant composition comprising a natural, organic antiperspirant active, in particular such a composition not comprising any industrially synthesised antiperspirant active.

The present disclosure provides the non-therapeutic use of benzaldehyde, honokiol or capsaicin as an antiperspirant agent.

In a second aspect of the invention, there is provided a cosmetic method of reducing perspiration comprising the topical application of a composition comprising a non-pore blocking antiperspirant agent selected from the group consisting of benzaldehyde, honokiol and capsaicin.

In a third aspect of the invention, there is provided an antiperspirant composition comprising a non-pore blocking antiperspirant agent selected from the group consisting of benzaldehyde, honokiol and capsaicin; a cosmetically acceptable carrier fluid; and a preservative and/or deodorant active.

In a fourth aspect of the invention, there is provided an antiperspirant product comprising a composition as described in the third aspect of the invention and a dispenser for said composition, the dispenser comprising containment means and application means for the composition.

In the cosmetic method described in the second aspect of the invention, the composition preferably comprises a cosmetically acceptable carrier fluid and a preservative and/or deodorant active.

### Detailed Description

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, all percentages, ratios and amounts are by weight, unless otherwise indicated.

Herein, all percentages, ratios and amounts are to be understood to be modified by the word "about" where appropriate.

Herein, the word "comprising" is intended to mean "including" but not necessarily "consisting of", i.e., it is non-exhaustive.

Herein, cosmetic methods are to be understood as a non-therapeutic in nature.

Herein, references to an amount of a compound or an active refer to the total amount of compounds or actives of the type indicated.

Herein, "application" and "applied" relate to application to the surface of the human body, in particular the underarm regions, unless the context dictates otherwise.

The invention typically involves the topical application of the non-pore blocking antiperspirant agent to the underarm regions of the human body, otherwise known as the axillae. From there, it is believed that the antiperspirant agent permeates to the secretory coil of the sweat glands. Without wishing to be bound by theory, it is believed that the antiperspirant agent reduces sweat production at its source, the sweat glands themselves.

The non-pore blocking antiperspirant agent is selected from the group consisting of benzaldehyde, honokiol and capsaicin. All of these are natural, organic compounds derivable from natural sources.

Benzaldehyde is a simple aromatic aldehyde having an almond odour. It may be derived from bitter almond oil.

Honokiol is an aromatic bisphenol compound. It may be derived from magnolia bark.

Capsaicin is an aromatic amide compound. It may be derived from chilli peppers and it is the principal component giving chilli peppers their hot sensation.

The non-pore blocking antiperspirant agent is preferably used from a composition comprising it at a level of from 0.01 to 10%, preferably from 0.02 to 5% and more preferably from 0.05 to 2%.

Compositions used in accordance with the present invention may optionally include pore-blocking antiperspirant agents, including conventional astringent aluminium-containing antiperspirant salts.

Such conventional astringent aluminium-containing antiperspirant salts include aluminium, zirconium and mixed aluminium/ zirconium salts, including both inorganic salts, salts with organic anions and complexes. Preferred astringent salts include aluminium, zirconium and aluminium/zirconium halides and halohydrate salts, such as chlorohydrates. Especially preferred aluminium halohydrate salts, known as activated aluminium chlorohydrates, are described in EP 6,739 (Unilever PLC and NV). Zirconium aluminium chlorohydrate actives are also preferred materials, as are the so-called ZAG (zirconium-aluminium-glycine) complexes, for example those disclosed in US 3,792,068 (Procter and Gamble Co.). Zinc phenol sulphonate may also be used, preferably at up to 3% by weight of the composition.

When employed, preferred levels of incorporation of conventional astringent aluminium-containing antiperspirant salts are from 0.5% to 60%, particularly from 5% to 40%, and especially from 5% or 10% to 30% or 35% of the composition.

In certain preferred embodiments of the invention, conventional astringent aluminium-containing antiperspirant salts are not employed.

The carrier fluid used in conjunction with the present invention must be cosmetically acceptable and it generally aids in the delivery of the non-pore blocking antiperspirant agent to the surface of the human body. The non-pore blocking antiperspirant agent is typically suspended or dissolved in the carrier fluid.

The carrier fluid may comprise from 10 to 99% of the composition.

In preferred aspects of the present invention, the carrier fluid for the non-pore blocking antiperspirant agent comprises a C₂-C₆ vicinal diol.

Herein, C₂-C₆ vicinal diols are compounds with from 2 to 6 carbon atoms in which two neighbouring carbon atoms bear hydroxyl groups.

Examples of C₂-C₆ vicinal diols include ethylene glycol, propylene glycol, glycerol and hexane-1,2-diol.

Preferred C₂-C₆ vicinal diols exclude compounds comprising more than 3 hydroxyl groups.

Preferred C₂-C₆ vicinal diols are C₂-C₄ vicinal diol, i.e. compounds with from 2 to 4 carbon atoms in which two neighbouring carbon atoms bear hydroxyl groups.

When employed, the C₂-C₆ vicinal diol in compositions of the invention is preferably present at from 1 to 50%, more preferably from 2 to 40% and most preferably from 5 to 25%.

A preferred additional component of the carrier fluid is ethanol. This may comprise up to 80% of the total composition, but is typically restricted to 70%, and often less than 60%. When employed ethanol typically comprises at least 10%, preferably at least 20%, and more preferably at least 40% by weight of total composition. Each of these preferred minimum levels of ethanol may be limited by the aforementioned maximum levels of ethanol.

A preferred additional component of the carrier fluid is water, especially when employed in conjunction with ethanol, to give an aqueous ethanol carrier fluid. Water may comprise up to 90% of the total composition, but is typically restricted to less than 60%, and often less than 50%. When employed water typically comprises at least 10%, preferably at least 20%, and more preferably at least 30% by weight of total composition. Each of these preferred minimum levels of water may be limited by the aforementioned maximum levels of water.

In the third aspect of the invention, the compositions comprise a preservative and/or deodorant active. Preservatives help to prevent microbial contamination of the composition. They are particularly important when the composition does not comprise a conventional, astringent aluminium containing antiperspirant salt.

Deodorant actives help counter malodour development on the surface of the human body. They are particularly important when the composition does not comprise a conventional, astringent aluminium containing antiperspirant salt.

The preservative and/or deodorant active is preferably an organic anti-microbial agent. Such agents may be selected from any of those known in the art, provided reasonable skill is used to avoid any incompatibilities. An organic anti-microbial agent is a particularly preferred additional component when the composition used does not include a conventional, astringent aluminium containing antiperspirant salt.

The preservative and/or deodorant active is typically used at a level of from 0.01 to 5% by weight of the composition.

Preferred anti-microbial deodorant agents are those that are more efficacious than simple alcohols such as ethanol. Particularly preferred anti-microbial deodorant agents are soluble in ethanol, meaning that they a solubility in ethanol of at least 10g/L at 20°C. Examples of suitable anti-microbial deodorant agents include niacinamide; quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S. A. Makin and M. R. Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals, 2',4,4'-trichloro,2-hydroxy-diphenyl ether (triclosan), 3,7,11-trimethyldodeca-2,6,10-trienol (farnesol), essential oils such as tea tree oil and thyme oil, climbazole, octapyrox, ketoconazole, zinc pyrithione and mixtures thereof.

A preferred optional component of compositions of the invention is a fragrance, typically at a level of from 0.1 to 5% of the total composition. In compositions also comprising water, the fragrance is preferably accompanied by a fragrance solubiliser, typically a nonionic surfactant used a concentration of from 0.1 to 5% of the total composition.

Thickening agents may be employed in compositions of the invention. Such agents increase the viscosity of or solidify the carrier fluid in which the antiperspirant agent is typically suspended or dissolved.

Thickening agents may be selected from any of those known in the art, provided reasonable skill is used to avoid any incompatibilities. A preferred class of thickeners, especially for compositions also comprising water, are hydroxyalkyl celluloses, such as hydroxypropyl cellulose. When employed, thickening agents are typically used at a level of from 0.1 to 40%. When a hydroxyalkyl cellulose thickening agent is employed, this is typically used at a level of from 0.2 to 10% by weight.

Dispensers suitable for use with the present invention comprise containment means and application means for the composition. They also typically comprise means for getting the composition from its containment means to its application means.

The containment means is preferably a reservoir for the composition and the application means is preferably a surface from which the composition may be massaged into the skin.

Preferred dispensers are able to function without the consumer needing to touch the composition with his or her hands in order for it to be applied, this feature enhancing dosing accuracy.

### Examples

The compositions detailed in Table 1 may be prepared by methods known in the art. The pH of the resulting composition is adjusted to 6 to 7 with 0.2N sodium hydroxide solution. The level of hydroxypropyl cellulose may be adjusted to gain the desired level of viscosity for the composition.

**Table 1**

| | **Example** | | | | |
|---|---|---|---|---|---|
| | **1** | **2** | **3** | **4** | **5** |
| Benzaldehyde | 1.0 | 0.5 | -- | -- | -- |
| Honokiol | -- | -- | 0.5 | 0.05 | -- |
| Capsaicin | -- | -- | -- | -- | 1.0 |
| Hydroxypropyl cellulose | 1.0 | 1.5 | 1.0 | 1.5 | 1.0 |
| Propylene glycol | 10.0 | -- | 12.0 | -- | 8.0 |
| Glycerol | 1.0 | -- | 0.8 | -- | 1.2 |
| Ethanol | 60 | 68.6 | 60 | 68.9 | 50 |
| Water | To 100 | To 100 | To 100 | To 100 | To 100 |

## Claims

1. The non-therapeutic use of benzaldehyde, honokiol or capsaicin as an antiperspirant agent.

2. The use according to claim 1, wherein the antiperspirant agent is topically applied to the surface of the human body as part of an antiperspirant composition.

3. The use according to claim 1 or claim 2, wherein a conventional astringent aluminium-containing antiperspirant salt is not also applied.

4. The use according to any of the preceding claims, wherein the antiperspirant agent is naturally-derived.

## Patentansprüche

1. Nicht-therapeutische Verwendung von Benzaldehyd, Honokiol oder Capsaicin als schweißhemmendes Mittel.

2. Verwendung nach Anspruch 1, wobei das schweißhemmende Mittel als Teil einer schweißhemmenden Zusammensetzung topisch auf die Oberfläche des menschlichen Körpers aufgetragen wird.

3. Verwendung nach Anspruch 1 oder Anspruch 2, wobei ein herkömmliches astringierendes Aluminium-haltiges schweißhemmendes Salz nicht mit aufgetragen wird.

4. Verwendung nach irgendeinem der vorhergehenden Ansprüche, wobei das schweißhemmende Mittel natürlichen Ursprungs ist.

## Revendications

1. Utilisation non-thérapeutique de benzaldéhyde, honokiol ou capsaïcine comme un agent antiperspirant.

2. Utilisation selon la revendication 1, dans laquelle l'agent antiperspirant est appliqué de manière topique sur la surface du corps humain comme une partie d'une composition antiperspirante.

3. Utilisation selon la revendication 1 ou revendication 2, dans laquelle un sel antiperspirant contenant de l'aluminium astringent classique n'est également pas appliqué.

4. Utilisation selon l'une quelconque des revendications précédentes, dans laquelle l'agent antiperspirant est dérivé d'une source naturelle.
